# EUROPÄISCHE PATENTSCHRIFT

(11) **EP 1 294 668 B1**
(45) Veröffentlichungstag und Bekanntmachung des Hinweises auf die Patenterteilung: **22.02.2006**
(21) Anmeldenummer: 01960406.5
(22) Anmeldetag: 27.06.2001
(51) Int. Cl.: C07C 45/50, C07C 47/02

(54) **VERFAHREN ZUR HYDROFORMYLIERUNG VON OLEFINEN MIT 2 BIS 8 KOHLENSTOFFATOMEN**
METHOD FOR THE HYDROFORMYLATION OF OLEFINS COMPRISING 2 TO 8 CARBON ATOMS
PROCEDE D'HYDROFORMYLATION D'OLEFINES AYANT 2 A 8 ATOMES DE CARBONE

(30) Priorität: 28.06.2000 DE 10031519
(43) Veröffentlichungstag der Anmeldung: 26.03.2003
(73) Patentinhaber: BASF Aktiengesellschaft, 67056 Ludwigshafen (DE)
(72) Erfinder: WALZ, Helmut, 67069 Ludwigshafen (DE); SCHÖNMANN, Willi, 67117 Limburgerhof (DE); MÜLLER, Rolf, 67125 Dannstadt-Schauernheim (DE); KROKOSZINSKI, Roland, 67273 Weisenheim a. Berg (DE)
(74) Vertreter: Kinzebach, Werner
(86) Internationale Anmeldenummer: PCT/EP2001/007342
(87) Internationale Veröffentlichungsnummer: WO 2002/000583

(56) Entgegenhaltungen:
- EP-A- 0 254 180
- EP-A- 0 648 730
- DE-A- 19 530 698
- GB-A- 2 055 367

## Beschreibung

Die vorliegende Erfindung betrifft ein Verfahren zur Hydroformylierung von Olefinen mit 2 bis 8 Kohlenstoffatomen.

Die Hydroformylierung oder Oxo-Synthese ist ein wichtiges großtechnisches Verfahren zur Herstellung von Aldehyden aus Olefinen, Kohlenmonoxid und Wasserstoff. Diese Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, speziell Co-, Rh-, Ir-, Pd-, Pt- oder Ru-Verbindungen bzw. -komplexe eingesetzt, die unmodifiziert oder z. B. mit amin- oder phosphinhaltigen Verbindungen modifiziert sein können.

Da ein quantitativer Umsatz des Olefins bei der Hydroformylierung einen hohen apparativen Aufwand erfordert, wird insbesondere die Hydroformylierung niederer Olefine häufig nur bis zu einem Teilumsatz durchgeführt. Aus dem Reaktionsaustrag wird das Hydroformylierungsprodukt abgetrennt und das nicht umgesetzte Olefin wird dann zusammen mit frischem Kohlenmonoxid und Wasserstoff wieder dem Hydroformylierungsreaktor zugeführt. Mit dem zurückgeführten Olefin werden allerdings auch die mit dem Olefin eingeführten oder durch Nebenreaktionen gebildeten Inertkomponenten, z. B. gesättigte Kohlenwasserstoffe, die der Hydroformylierung nicht zugänglich sind, in den Reaktor zurückgeführt. Um zu verhindern, dass die Konzentration der Inertkomponenten im Hydroformylierungsreaktor kontinuierlich ansteigt und Werte erreicht, bei denen die Hydroformylierungsreaktion zum Erliegen kommt, muss kontinuierlich ein Teilstrom des rückgeführten Stroms aus dem Verfahren ausgeleitet werden, um die Inertkomponenten aus dem System zu entfernen.

Der Ausleitstrom besteht jedoch nur zu einem Teil aus Inertkomponenten. Den größten Teil bilden nicht umgesetztes Olefin sowie nicht umgesetztes Kohlenmonoxid und Wasserstoff, die somit für die Umsetzung verloren gehen. Um den Ausleitstrom klein und die damit verbundenen Verluste gering zu halten, wird im Allgemeinen ein Olefinzulauf hoher Reinheit eingesetzt. So wird bei der Hydroformylierung von Propylen im Allgemeinen ein Propylenzulauf mit einer Reinheit von etwa 99,5 % eingesetzt, das bisweilen als "Polymer Grade Propylen" bezeichnet wird, wobei der Rest im Wesentlichen aus Propan besteht. Derartige Olefinzuläufe hoher Reinheit sind allerdings nur aufwendig zu erhalten und werden demzufolge zu deutlich höheren Preisen auf dem Markt angeboten als Olefine niedrigerer Reinheit. So ist beispielsweise das sogenannte "Chemical Grade Propylen", das etwa 3 bis 7 Gew.-% Propan enthält, deutlich billiger als das angesprochene Polymer Grade Propylen.

Olefinzuläufe niedriger Reinheit, die z. B. mehr als 0,5 Gew.-% gesättigte Kohlenwasserstoffe enthalten, können in industriellen Hydroformylierungsverfahren aus den genannten Gründen nicht ohne weiteres eingesetzt werden. Um zu verhindern, dass die Konzentration gesättigter Kohlenwasserstoffe im Hydroformylierungsreaktor derart hohe Werte erreicht, dass die Hydroformylierung zum Erliegen kommt, müsste der Ausleitstrom unter gleichzeitigem Verlust von unumgesetztem Olefin derart hoch gewählt werden, dass die Ersparnis des billigeren Einsatzstoffs zunichte gemacht würde.

Die EP-A 0 648 730 offenbart ein Verfahren zur Herstellung eines aus Propylen gewonnenen Oxoprodukts, bei dem aus dem Produktstrom einer Propylen-Hydroformylierung ein Gasstrom abgetrennt wird, der nicht umgesetztes Propylen und Propan enthält. Durch selektive Adsorption von Propylen an ein Adsorptionsmittel und anschließende Desorption wird ein Propylen-angereicherter Gasstrom erhalten, der zumindest teilweise in die Reaktionszone zurückgeführt wird. Die alternierenden Adsorptions- und Desorptionscyclen erfordern periodische Druck- und/oder Temperaturwechsel. Die dazu benötigten Apparaturen sind aufwendig und störanfällig.

Die Aufgabe der vorliegenden Erfindung besteht daher darin, ein alternatives Verfahren zur Hydroformylierung von Olefinen anzugeben, das den Einsatz von olefinhaltigen Zuläufen mit einer Beimischung von gesättigten Kohlenwasserstoffen erlaubt.

Diese Aufgabe wird erfindungsgemäß durch ein Verfahren zur Hydroformylierung von Olefinen mit 2 bis 8 Kohlenstoffatomen gelöst, bei dem man
(i) in eine Reaktionszone einen olefinhaltigen Zulauf, der eine Beimischung eines gesättigten Kohlenwasserstoffs mit 2 bis 8 Kohlenstoffatomen enthält, Kohlenmonoxid und Wasserstoff einspeist und in Gegenwart eines Hydroformylierungskatalysators, umsetzt,
(ii) vom Austrag aus der Reaktionszone einen im Wesentlichen aus nicht umgesetztem Olefin und gesättigtem Kohlenwasserstoff bestehenden Strom abtrennt,
(iii) den Strom durch Rektifikation in eine Olefin-angereicherte Fraktion und eine Olefin-abgereicherte Fraktion trennt, und
(iv) die Olefin-angereicherte Fraktion zumindest teilweise in die Reaktionszone zurückführt.

Olefine, die nach dem erfindungsgemäßen Verfahren hydroformyliert werden können, enthalten 2 bis 8, vorzugsweise 2 bis 4 Kohlenstoffatöme. Es kann sich um geradkettige, verzweigte oder cyclische Olefine handeln. Bevorzugte Beispiele geeigneter Olefine sind Ethen, Propen, 1-Buten und/oder 2-Buten. Der olefinhaltige Zulauf kann ein einzelnes Olefin oder ein Gemisch von Olefinen enthalten. Das Olefin wird in Form eines olefinhaltigen Zulaufs in die Reaktionszone eingespeist, der eine Beimischung, z. B. 0,5 bis 40 Gew.-%, vorzugsweise 2 bis 30 Gew.-%, insbesondere 3 bis 10 Gew.-%, wenigstens eines gesättigten Kohlenwasserstoffs, d. h. eines Alkans und/oder Cycloalkans, mit 2 bis 8 Kohlenstoffatomen enthält. In der Regel weisen das Olefin und der gesättigte Kohlenwasserstoff die gleiche Anzahl an Kohlenstoffatomen auf. Üblicherweise besteht der olefinhaltige Zulauf im Wesentlichen nur aus Olefin und gesättigtem Kohlenwasserstoff, d. h. er enthält vorzugsweise weniger als 0,5 Gew.-% an von Olefin und gesättigtem Kohlenwasserstoff verschiedenen Komponenten.

Geeignete olefinhaltige Zuläufe sind großtechnisch zugänglich. Ein bevorzugtes Beispiel ist ein Gemisch von Propan und Propen, vorzugsweise mit einem Gehalt von 2 bis 10 Gew.-%, insbesondere 3 bis 7 Gew.-%, Propan. Derartige Gemische sind als "Chemical Grade Propylen" käuflich. Sie werden z. B. erhalten durch Umsetzung von Naptha oder Erdgas in Steamcrackern und anschließende destillative Aufarbeitung. Ein weiteres Beispiel eines geeigneten olefinhaltigen Zulaufs ist auch das sogenannte "Refinery Grade Propylen", das Propangehalte von 20 bis 40 Gew.-% aufweist.

Kohlenmonoxid und Wasserstoff werden üblicherweise in Form eines Gemisches, dem sogenannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel 2:1 bis 1:2, insbesondere etwa 45:55 bis 50:50.

Die Temperatur bei der Hydroformylierungsreaktion liegt im Allgemeinen in einem Bereich von etwa 50 bis 200 °C, vorzugsweise etwa 60 bis 190 °C, insbesondere etwa 90 bis 190 °C. Die Umsetzung wird vorzugsweise bei einem Druck im Bereich von etwa 10 bis 700 bar, vorzugsweise 15 bis 200 bar, insbesondere 15 bis 60 bar durchgeführt. Der Reaktionsdruck kann in Abhängigkeit von der Aktivität des eingesetzten Hydroformylierungskatalysators variiert werden.

Geeignete druckfeste Reaktionsapparaturen für die Hydroformylierung sind dem Fachmann bekannt. Dazu zählen die allgemein üblichen Reaktoren für Gas-flüssig-Reaktionen, wie z. B. Gasumlaufreaktoren, Blasensäulen etc., die gegebenenfalls durch Einbauten unterteilt sein können.

Geeignete Hydroformylierungskatalysatoren sind die üblichen, dem Fachmann bekannten Übergangsmetallverbindungen und -komplexe, die sowohl mit als auch ohne Cokatalysatoren eingesetzt werden können. Bevorzugt handelt es sich bei dem Übergangsmetall um ein Metall der VIII. Nebengruppe des Periodensystems und insbesondere um Co, Ru, Rh, Pd, Pt, Os oder Ir, speziell um Rh, Co, Ir oder Ru.

Geeignete Komplexverbindungen sind beispielsweise die Carbonylverbindungen der genannten Metalle sowie Komplexe, deren Liganden ausgewählt sind unter Aminen, Arylphosphinen, Alkylphosphinen, Arylalkylphosphinen, Olefinen, Dienen etc. und Mischungen davon.

Es eignen sich z. B. Rhodiumkomplexe der allgemeinen Formel RhXₘL¹L² (L³)ₙ, worin
- X: für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion,
- L¹, L², L³: unabhängig voneinander für CO, Olefine, Cycloolefine, vorzugsweise Cyclooctadien (COD), Dibenzophosphol, Benzonitril, PR₃ oder R₂P-A-PR₂, m für 1 oder 3 und n für 0, 1 oder 2 stehen. Unter R (die Reste R können gleich oder verschieden sein) sind Alkyl-, Cycloalkyl- und Arylreste zu verstehen, vorzugsweise Phenyl, p-Tolyl, m-Tolyl, p-Ethylphenyl, p-Cumyl, p-t-Butylphenyl, p-C₁-C₄-Alkoxyphenyl, vorzugsweise p-Anisyl, Xylyl, Mesityl, p-Hydroxyphenyl, das gegebenenfalls auch ethoxyliert vorliegen kann, Sulfophenyl, Isopropyl, C₁-C₄-Alkoxy, Cyclopentyl oder Cyclohexyl. A steht für 1,2-Ethylen oder 1,3-Propylen. Bevorzugt stehen L¹, L² oder L³ unabhängig voneinander für CO, COD, P(Phenyl)₃, P(i-Propyl)₃, P(Anisyl)₃, P(OC₂H₅)₃, P(Cyclohexyl)₃, Dibenzophosphol oder Benzonitril.
- X: steht bevorzugt für Hydrid, Chlorid, Bromid, Acetat, Tosylat, Acetylacetonat oder das Diphenyltriazin-Anion, insbesondere für Hydrid, Chlorid oder Acetat.

Bevorzugte Hydroformylierungskatalysatoren sind phosphorhaltige Rhodiumkatalysatoren, wie sie z. B. unter Hydroformylierungsbedingungen in situ aus einer Rhodiumquelle und einem Triarylphosphin, z. B. Triphenylphosphin, gebildet werden, wie RhH(CO)₂(PPh₃)₂ oder RhH(CO)(PPh₃)₃.

Geeignete Hydroformylierungskatalysatoren werden z. B. in Beller et al., Journal of Molecular Catalysis A, 104 (1995), S. 17-85, beschrieben, worauf hier in vollem Umfang Bezug genommen wird.

In der Reaktionszone findet, bezogen auf das eingespeiste Olefin, pro Durchgang ein Teilumsatz statt. Der Umsatz beträgt im Allgemeinen 10 bis 90 % bezogen auf das eingespeiste Olefin.

Der Austrag aus der Reaktionszone wird einer ein- oder mehrstufigen Trennoperation unterzogen, wobei zumindest ein die Hauptmenge des Hydroformylierungsproduktes enthaltender Strom und ein im Wesentlichen aus nicht umgesetztem Olefin und gesättigtem Kohlenwasserstoff bestehender Strom erhalten werden. Je nach Austragsverfahren werden gegebenenfalls weitere Ströme erhalten, wie synthesegashaltige Abgase, hochsiedende Nebenprodukte der Hydroformylierung und/oder Hydroformylierungskatalysator enthaltende Ströme, die - gegebenenfalls nach Aufarbeitung - ganz oder teilweise in die Reaktionszone zurückgeführt oder aus dem Verfahren ausgeschleust werden. Vom Austrag aus der Reaktionszone können beispielsweise zuerst das Hydroformylierungsprodukt und gegebenenfalls höher als das Hydroformylierungsprodukt siedende Anteile abgetrennt werden. Anschließend kann ein Gemisch von nicht umgesetztem Olefin und gesättigtem Kohlenwasserstoff auskondensiert werden.

Zweckmäßigerweise erhält man den im Wesentlichen aus nicht umgesetztem Olefin und gesättigtem Kohlenwasserstoff bestehenden Strom jedoch dadurch, dass man vom Austrag aus der Reaktionszone zuerst ein rohes Hydroformylierungsprodukt abtrennt, welches nicht umgesetztes Olefin und gesättigten Kohlenwasserstoff gelöst enthält, und das flüssige rohe Hydroformylierungsprodukt dann einem Entgasungsschritt unterzieht, bei dem ein Strom anfällt, der im Wesentlichen aus nicht umgesetztem Olefin und gesättigtem Kohlenwasserstoff besteht. Der vom rohen Hydroformylierungsprodukt befreite Reaktionsaustrag wird in der Regel ganz oder teilweise in die Reaktionszone zurückgeführt. Zur Entgasung kann das rohe Hydroformylierungsprodukt entspannt, erwärmt und/oder mit einem Strippgas, wie Synthesegas oder Stickstoff, behandelt werden. Zweckmäßigerweise führt man die Entgasung in einer Kolonne durch, wobei das rohe Hydroformylierungsprodukt im Bereich der Kolonnenmitte eingespeist wird, am Kolonnensumpf das entgaste Hydroformylierungsprodukt abgezogen wird, das der weiteren Aufarbeitung zugeführt wird, und am Kolonnenkopf ein flüssiger oder gasförmiger Strom abgezogen wird, der im Wesentlichen aus nicht umgesetztem Olefin und gesättigtem Kohlenwasserstoff besteht.

Die Abtrennung des rohen Hydroformylierungsproduktes vom Austrag aus der Reaktionszone kann auf verschiedene Weise erfolgen. Es kann zum einen das sogenannte Flüssigaustragsverfahren zum Einsatz kommen, bei dem der im Wesentlichen, bis auf das im Überschuss zur Hydroformylierung eingesetzte Synthesegas, flüssige Austrag aus der Reaktionszone entspannt wird, wobei in Folge der Druckerniedrigung der Austrag in eine Flüssigphase, die im Wesentlichen aus hochsiedenden Nebenprodukten, dem homogen gelösten Hydroformylierungskatalysator und geringen Mengen an Hydroformylierungsprodukt, an nicht umgesetztem Olefin und an gesättigtem Kohlenwasserstoff besteht, und eine Gasphase, die im Wesentlichen aus Hydroformylierungsprodukt, nicht umgesetztem Olefin und gesättigtem Kohlenwasserstoff sowie nicht umgesetztem Synthesegas besteht, aufgetrennt wird. Die Flüssigphase kann als Rückführstrom wieder in den Reaktor geleitet werden. Durch zumindest partielle Kondensation der Gasphase wird das rohe Bydroformylierungsprodukt erhalten. Die bei der Kondensation zurückbleibende Gasphase wird ganz oder teilweise in die Reaktionszone zurückgeführt.

Mit Vorteil kann die in der Entspannungsstufe primär anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Flüssigphase und Gasphase werden dadurch im Gegenstrom geführt. Um den gegenseitigen Kontakt der Phasen zu erhöhen, ist die Kolonne vorzugsweise mit Füllkörpern gefüllt. Durch den innigen Kontakt der Gasphase mit der Flüssigphase werden die in der Flüssigphase vorhandenen Restmengen an Hydroformylierungsprodukt, nicht umgesetztem Olefin und gesättigtem Kohlenwasserstoff in die Gasphase transferiert, so dass der die Kolonne am Kopf verlassende Gasstrom im Vergleich zu dem am unteren Ende der Kolonne eingeführten Gasstrom an Hydroformylierungsprodukt, nicht umgesetztem Olefin und gesättigtem Kohlenwasserstoff angereichert ist. Die weitere Aufarbeitung des die Kolonne verlassenden Gasstroms und der die Kolonne verlassenden Flüssigphase erfolgt in üblicher Weise, beispielsweise wie oben beschrieben.

Alternativ kann man insbesondere bei Einsatz von C₂-C₄-Olefinen nach dem sogenannten Kreisgasverfahren (gas recycle) arbeiten, bei dem aus dem Gasraum des Hydroformylierungsreaktors ein Gasstrom abgezogen wird. Dieser Gasstrom besteht im Wesentlichen aus Synthesegas, nicht umgesetztem Olefin und gesättigtem Kohlenwasserstoff, wobei nach Maßgabe des Dampfdrucks im Hydroformylierungsreaktor das bei der Hydroformylierungsreaktion gebildete Hydroformylierungsprodukt mitgeführt wird. Aus dem Gasstrom wird das mitgeführte Hydroformylierungsprodukt z. B. durch Abkühlen auskondensiert, und der vom Flüssiganteil befreite Gasstrom wird zurück in den Hydroformylierungsreaktor geführt.

Der im Wesentlichen aus nicht umgesetztem Olefin und gesättigtem Kohlenwasserstoff bestehende Strom enthält z. B. 50 bis 95 Gew.-%, vorzugsweise 60 bis 80 Gew.-%, Olefin und 5 bis 50 Gew.-%, vorzugsweise 20 bis 40 Gew.-% gesättigten Kohlenwasserstoff.

Der im wesentlichen aus nicht umgesetztem Olefin und gesättigtem Kohlenwasserstoff bestehende Strom wird durch Rektifikation (Destillation) in eine olefin-angereicherte Fraktion und eine Olefin-abgereicherte Fraktion getrennt. Die Rektifikation erfolgt üblicherweise bei tiefer Temperatur und/oder erhöhtem Druck, wobei die genauen Temperatur- und/oder Druckbedingungen von Faktoren, wie der Kohlenstoffanzahl des zu trennenden Olefins/gesättigten Kohlenwasserstoffs, usw. abhängen. Die Rektifikation erfolgt im Allgemeinen in einer Kolonne, die mit einer ausreichend großen Zahl von Rektifikationsböden versehen ist. Kolonnen für derartige Trennaufgaben sind an sich bekannt und werden z. B. zur Trennung von Olefinen und gesättigten Kohlenwasserstoffen eingesetzt, die im Spaltgas eines Steamcrackers enthalten sind. Der aufzutrennende Strom kann wahlweise gasförmig oder flüssig in die Kolonne eingeführt werden, vorzugsweise im Bereich der Kolonnenmitte. Am Kopf oder oberen Bereich der Kolonne kann zweckmäßigerweise die Olefin-angereicherte Fraktion, am Sumpf oder unteren Bereich der Kolonne die Olefin-abgereicherte Fraktion abgenommen werden.

In der Regel strebt man an, als Olefin-abgereicherte Fraktion möglichst reinen gesättigten Kohlenwasserstoff zu gewinnen, der ohne größeren Verlust an Olefin aus dem Verfahren ausgeleitet werden kann. Demgegenüber strebt man bei der Olefin-angereicherten Fraktion im Allgemeinen nicht reines Olefin an, sondern lässt darin einen gewissen Gehalt an gesättigten Kohlenwasserstoffen zu, um den Aufwand der Trennung geringer zu halten. Es ist für die Zwecke der vorliegenden Erfindung ausreichend, dass die Olefin-angereicherte Fraktion gegenüber dem Austrag aus der Reaktionszone Olefin-angereichert ist, d. h. dass das Verhältnis von Olefin zu gesättigtem Kohlenwasserstoff in ihr größer ist als im Austrag aus der Reaktionszone.

Die Olefin-abgereicherte Fraktion enthält vorzugsweise mehr als 95 Gew.-%, insbesondere mehr als 99 Gew.-% gesättigten Kohlenwasserstoff. Üblicherweise enthält die Olefin-angereicherte Fraktion mehr als 80 Gew.-%, z. B. 85 bis 95 Gew.-% Olefin, wobei der Rest gesättigter Kohlenwasserstoff ist.

Nach einer besonders bevorzugten Ausführungsform wird im erfindungsgemäßen Verfahren ein propylenhaltiger Zulauf eingesetzt, der eine Beimischung von Propan enthält. Als zu trennender Strom fällt daher ein Gemisch von Propylen und Propan an. Die Auftrennung dieses Stroms in eine Propylen-angereicherte Fraktion und eine propylen-abgereicherte Fraktion gelingt in einer geeigneten Destillationskolonne unter Druck, einem sogenannten C₃-Splitter. Die Kolonne wird vorzugsweise so gefahren, dass am Kopf eine Propylen-angereicherte Fraktion anfällt, die direkt in die Reaktionszone zurückgeführt werden kann, und am Sumpf weitgehend reines Propan abgezogen werden kann, das ohne Verlust an Propylen aus dem System entfernt werden kann. Typische Betriebsbedingungen des C₃-Splitters sind: 20 bis 25 bar Kopfdruck, 60 bis 70°C Sumpftemperatur, 100 bis 150 theoretische Böden.

Die Olefin-abgereicherte Fraktion wird aus dem System ausgeleitet. Sie kann z. B. verbrannt werden. Sie kann ferner als Einsatzstoff für chemische Umsetzungen, z. B. im Steamcracker, dienen. In einer besonders zweckmäßigen Ausführungsform wird die olefin-abgereicherte Fraktion einem Steamcracker zugeführt, aus dessen Spaltgas der olefinhaltige Zulauf für das erfindungsgemäße Verfahren gewonnen wird. Zur Erreichung eines stationären Betriebszustandes des erfindungsgemäßen Verfahrens wird mit der Olefin-abgereicherten Fraktion eine Menge an gesättigtem Kohlenwasserstoff ausgeleitet, die im Wesentlichen der Summe der mit dem olefinhaltigen Zulauf eingeführten Menge und der bei der Hydroformylierung gebildeten Menge an gesättigtem Kohlenwasserstoff entspricht.

Das erfindungsgemäße Verfahren geht von olefinhaltigen Zuläufen aus, die eine Beimischung eines gesättigten Kohlenwasserstoffs enthalten. Derartige Gemische fallen großtechnisch an und ihre Gewinnung ist erheblich weniger aufwendig als die der entsprechenden hochgereinigten Olefinzuläufe. Im Rahmen der Erfindung ist zwar an einer der Hydroformylierung nachgeschalteten Stelle eine Auftrennung von Olefin und gesättigtem Kohlenwasserstoff erforderlich. Die Auftrennung eines vom Hydroformylierungsaustrag abgetrennten Stroms von Olefin/gesättigtem Kohlenwasserstoff ist jedoch deutlich weniger aufwendig ist als die Auftrennung des gesamten olefinhaltigen Zulaufs, da der nach der Hydroformylierung anfallende Strom um den Olefinanteil verringert ist, der in der Hydroformylierungsreaktion zum Aldehyd und/oder Alkohol umgesetzt worden ist. Die anfallenden Mengen an aufzutrennendem Gemisch sind in der der Reaktionszone nachgeschalteten Stufe erheblich geringer als der vollständige Zulauf. Somit kann die Trennungsanlage kleiner ausgelegt werden, was mit geringeren Investitionskosten verbunden ist. Ein weiterer Vorteil ist darin zu sehen, dass der Olefin-Gehalt der Olefin-angereicherten Fraktion lediglich soweit erhöht werden muss, dass das Gewichtsverhältnis von Olefin zu gesättigtem Kohlenwasserstoff in der Olefin-angereicherten Fraktion größer ist als im Austrag aus der Reaktionszone. Ein olefinhaltiger Zulauf in herkömmlichen Hydroformylierungsverfahren weist in der Regel deutlich höhere Olefingehalte, z. B. etwa 99,5 %, auf.

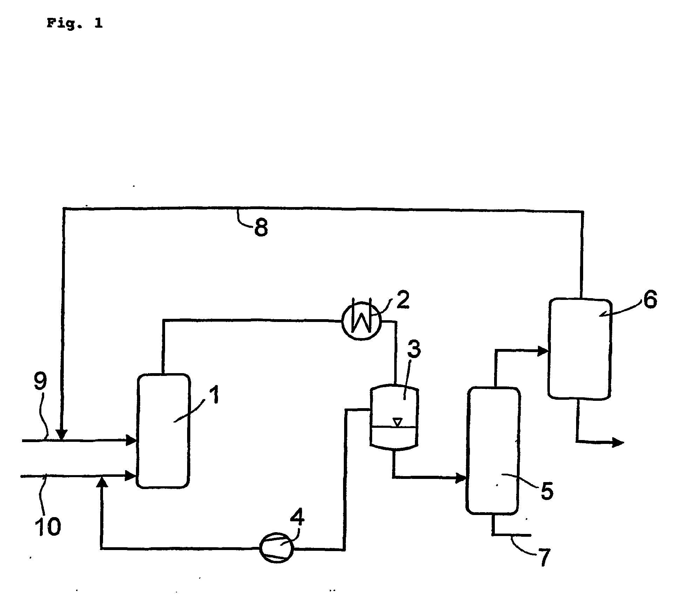

Eine vorteilhafte Ausgestaltung des erfindungsgemäßen Verfahrens ist in Fig. 1 dargestellt und wird im Folgenden erläutert.

Fig.1 zeigt eine schematische Darstellung des erfindungsgemäßen Verfahrens nach dem Kreisgasverfahren. An sich selbstverständliche Anlagendetails, die zur Veranschaulichung des erfindungsgemäßen Verfahrens nicht erforderlich sind, wurden aus Gründen der Übersichtlichkeit weggelassen.

Gemäß Fig. 1 wird in den Reaktor (1) ein olefinhaltiger Zulauf (9), der das zu hydroformylierende Olefin und einen gesättigten Kohlenwasserstoff enthält, und ein durch die Rohrleitung (8) zurückgeführter olefinhaltiger Strom sowie Synthesegas (10), d. h. ein Gemisch von Kohlenmonoxid und Wasserstoff, eingespeist und dort bis zu einem Teilumsatz hydroformyliert. Aus dem Gasraum des Reaktors wird ein gasförmiger Strom entnommen, der nicht umgesetztes Olefin, gesättigten Kohlenwasserstoff, nicht umgesetztes Synthesegas und ein Hydroformylierungsprodukt enthält. Der Strom wird im Wärmetauscher (2) gekühlt und einem Phasentrenngefäß (3) zugeführt. Der gasförmige Anteil wird über den Kompressor (4) wieder dem Reaktor (1) zugeführt. Der im Trenngefäß (3) anfallende flüssige Anteil, der im Wesentlichen aus rohem Hydroformylierungsprodukt mit darin gelöstem Olefin und gesättigtem Kohlenwasserstoff besteht, wird der Entgasungskolonne (5) zugeführt, an deren Kopf ein Gemisch von Olefin und gesättigtem Kohlenwasserstoff anfällt. Im Sumpf der Entgasungskolonne (5) wird das rohe Hydroformylierungsprodukt (7) entnommen, das anschließend weiter aufgearbeitet wird. Das Gemisch von Olefin und gesättigtem Kohlenwasserstoff wird in die Rektifikationskolonne (6) geleitet. Dort fällt am Kopf ein Olefinstrom mit vermindertem Anteil an gesättigtem Kohlenwasserstoff an, der über die Rohrleitung (8) in den Hydroformylierungsreaktor (1) zurückgeführt wird. Am Sumpf der Rektifikationskolonne (6) erhält man im Wesentlichen reinen gesättigten Kohlenwasserstoff, der aus dem System entfernt wird.

Die Erfindung wird durch das folgende Beispiel näher veranschaulicht.

### Beispiel

Es wurde eine Anlage verwendet, wie sie in Fig. 1 dargestellt ist.

Dem Reaktor (1) wurden ein Zulauf von 10 t/h Chemical Grade Propylen (95 % Propylen, 5 % Propan), ein Rückführstrom (8) von 3,2 t/h aus der propylen-propan-Trennkolonne (6) sowie Synthesegas zugeführt. Im Reaktor befand sich eine Flüssigphase aus Hochsiedern und einem darin homogen gelösten Katalysator in Form von Rh/Triphenylphosphin. Die Hydroformylierung erfolgte bei 105 °C und 20 bar. Das gebildete Produkt, ein Gemisch aus n- und iso-Butyraldehyd, wurde zusammen mit nicht umgesetztem Propylen sowie dem zugeführten und dem gebildeten Propan mit Hilfe eines Kreisgasstroms aus dem Reaktor ausgetragen. Die kondensierbaren Anteile wurden in dem nachgeschalteten Kühler (2) niedergeschlagen und dem darauffolgenden Abscheider (3) gesammelt. Das Kondensat enthielt 78,3 % Butyraldehyd, 14,3 % Propylen und 7,4 % Propan. Es wurde der Entgasungskolonne (5) zugeführt (20,3 t/h), wo im Sumpf ein C₃-freies Hydroformylierungsprodukt (15,9 t/h) und am Kopf ein Gemisch aus 66 % Propylen und 34 % Propan (4,4 t/h) anfiel. Dieses Gemisch wurde in der nachfolgenden Kolonne (6) aufgetrennt in einen praktisch propylenfreien Propanstrom am Sumpf (1,2 t/h) und ein Gemisch aus 90 % Propylen und 10 % Propan am Kopf (3,2 t/h). Dieser Strom wurde wieder dem Propylenzulauf des Synthesereaktors (1) zugeführt. Die Kolonne (6) wurde bei einem Kopfdruck von 20 bis 21 bar und einer Sumpftemperatur von 60 bis 70 °C betrieben und wies 130 praktische Böden auf.

## Patentansprüche

1. Verfahren zur Hydroformylierung von Olefinen mit 2 bis 8 Kohlenstoffatomen, bei dem man
(i) in eine Reaktionszone einen olefinhaltigen Zulauf, der eine Beimischung eines gesättigten Kohlenwasserstoffs mit 2 bis 8 Kohlenstoffatomen enthält, sowie Kohlenmonoxid und Wasserstoff einspeist und in Gegenwart eines Hydroformylierungskatalysators umsetzt,
(ii) vom Austrag aus der Reaktionszone einen im Wesentlichen aus nicht umgesetztem Olefin und gesättigtem Kohlenwasserstoff bestehenden Strom abtrennt,
(iii) den Strom durch Rektifikation in eine Olefin-angereicherte Fraktion und eine Olefin-abgereicherte Fraktion trennt, und
(iv) die Olefin-angereicherte Fraktion zumindest teilweise in die Reaktionszone zurückführt.

2. Verfahren nach Anspruch 1, bei dem man den im Wesentlichen aus nicht umgesetztem Olefin und gesättigtem Kohlenwasserstoff bestehenden Strom **dadurch** erhält, dass man vom Austrag aus der Reaktionszone zuerst ein rohes Hydroformylierungsprodukt abtrennt, welches nicht umgesetztes Olefin und gesättigten Kohlenwasserstoff gelöst enthält, und das rohe Hydroformylierungsprodukt einem Entgasungsschritt unterzieht.

3. Verfahren nach Anspruch 2, bei dem der Austrag aus der Reaktionszone im Wesentlichen gasförmig ist, und das rohe Hydroformylierungsprodukt durch Kondensation aus dem gasförmigen Austrag abgetrennt wird.

4. Verfahren nach Anspruch 2, bei dem der Austrag aus der Reaktionszone im Wesentlichen flüssig ist, der flüssige Austrag entspannt wird, wobei eine Auftrennung in eine Flüssigphase, die im Wesentlichen aus hochsiedenden Nebenprodukten, dem homogen gelösten Hydroformylierungskatalysator und geringen Mengen an Hydroformylierungsprodukt, geringen Mengen an nicht umgesetztem Olefin und geringen Mengen an gesättigtem Kohlenwasserstoff besteht, und eine Gasphase, die im Wesentlichen aus Hydroformylierungsprodukt, nicht umgesetztem Olefin und gesättigtem Kohlenwasserstoff sowie nicht umgesetztem Kohlenmonoxid und Wasserstoff besteht, erfolgt, und das rohe Hydroformylierungsprodukt durch zumindest partielle Kondensation der Gasphase erhalten wird.

5. Verfahren nach einem der vorhergehenden Ansprüche, bei dem man als Hydroformylierungskatalysator einen phosphorhaltigen Rhodiumkatalysator verwendet.

6. Verfahren nach einem der vorhergehenden Ansprüche, bei dem das Olefin und der gesättigte Kohlenwasserstoff die gleiche Anzahl an Kohlenstoffatomen aufweisen.

7. Verfahren nach Anspruch 6, bei dem es sich bei dem olefinhaltigen Zulauf um ein Gemisch von Propan und Propylen handelt.

8. Verfahren nach Anspruch 7, bei dem das Gemisch 2 bis 10 Gew.-% Propan enthält.

9. Verfahren nach einem der vorhergehenden Ansprüche, bei dem die Olefin-abgereicherte Fraktion einem Steamcracker zugeführt wird.

## Claims

1. A process for the hydroformylation of olefins having from 2 to 8 carbon atoms, in which
(i) an olefin-comprising feed comprising a proportion of a saturated hydrocarbon having from 2 to 8 carbon atoms and also carbon monoxide and hydrogen are fed into a reaction zone and reacted in the presence of a hydroformylation catalyst,
(ii) a stream consisting essentially of unreacted olefin and saturated hydrocarbon is separated off from the output from the reaction zone,
(iii) the stream is separated into an olefin-enriched fraction and an olefin-depleted fraction by rectification, and
(iv) at least part of the olefin-enriched fraction is recirculated to the reaction zone.

2. The process according to claim 1, in which the stream consisting essentially of unreacted olefin and saturated hydrocarbon is obtained by firstly separating off a crude hydroformylation product comprising unreacted olefin and saturated hydrocarbon in dissolved form from the output from the reaction zone and subjecting the crude hydroformylation product to a degassing step.

3. The process according to claim 2, in which the output from the reaction zone is essentially gaseous and the crude hydroformylation product is separated off by condensation from the gaseous output.

4. The process according to claim 2, in which the output from the reaction zone is essentially liquid, the liquid output is depressurized so that it is separated into a liquid phase which consists essentially of high-boiling by-products, the homogeneously dissolved hydroformylation catalyst and small amounts of hydroformylation product, small amounts of unreacted olefin and small amounts of saturated hydrocarbon, and a gas phase which consists essentially of hydroformylation product, unreacted olefin and saturated hydrocarbon and also unreacted carbon monoxide and hydrogen, and the crude hydroformylation product is obtained by at least partial condensation of the gas phase.

5. The process according to any of the preceding claims, in which the hydroformylation catalyst used is a phosphorus-comprising rhodium catalyst.

6. The process according to any of the preceding claims, in which the olefin and the saturated hydrocarbon have the same number of carbon atoms.

7. The process according to claim 6, in which the olefin-comprising feed is a mixture of propane and propylene.

8. The process according to claim 7, in which the mixture comprises from 2 to 10% by weight of propane.

9. The process according to any of the preceding claims, in which the olefin-depleted fraction is fed to a steam cracker.

## Revendications

1. Procédé d'hydroformylation d'oléfines ayant 2 à 8 atomes de carbone, dans lequel :
(i) on introduit, dans une zone réactionnelle, une charge oléfinique qui contient un mélange d'un hydrocarbure saturé ayant 2 à 8 atomes de carbone, ainsi que du monoxyde de carbone et de l'hydrogène et qu'on fait réagir en présence d'un catalyseur d'hydroformylation;
(ii) on sépare de la décharge de la zone réactionnelle un flux constitué essentiellement d'oléfine non transformée et d'hydrocarbure saturé;
(iii) on sépare le flux par rectification dans une fraction enrichie en oléfine et une fraction appauvrie en oléfine, et
(iv) on refoule la fraction enrichie en oléfine au moins en partie dans la zone réactionnelle.

2. Procédé selon la revendication 1, dans lequel on obtient le flux constitué essentiellement d'oléfine non transformée et d'hydrocarbure saturé en séparant de la décharge de la zone réactionnelle, d'abord un produit d'hydroformylation brut, lequel contient, sous forme dissoute, une oléfine non transformée et un hydrocarbure saturé, et en soumettant le produit d'hydroformylation à une étape de dégazage.

3. Procédé selon la revendication 2, dans lequel la décharge de la zone réactionnelle est essentiellement sous forme gazeuse et le produit brut d'hydroformylation est séparé de la décharge sous forme gazeuse par condensation.

4. Procédé selon la revendication 2, dans lequel la décharge de la zone réactionnelle est essentiellement liquide, la sortie liquide est détendue, et il s'effectue à cette occasion une séparation en une phase liquide, laquelle est constituée essentiellement de produits secondaires à point d'ébullition élevé, du catalyseur d'hydroformylation dissous de manière homogène et de petites quantités de produit d'hydroformylation, de petites quantités d'oléfine non transformée et de petites quantités d'hydrocarbure saturé, et une phase gazeuse constituée essentiellement du produit d'hydroformylation, d'oléfine non transformée et d'hydrocarbure saturé ainsi que de monoxyde de carbone non transformé et d'hydrogène, et on obtient le produit brut d'hydroformylation par condensation au moins partielle de la phase gazeuse.

5. Procédé selon l'une quelconque des revendications précédentes, dans lequel on utilise comme catalyseur d'hydroformylation un catalyseur phosphoreux au rhodium.

6. Procédé selon l'une quelconque des revendications précédentes, dans lequel l'oléfine et l'hydrocarbure saturé présentent le même nombre d'atomes de carbone.

7. Procédé selon la revendication 6, dans lequel il s'agit, pour la charge contenant une oléfine, d'un mélange de propane et de propylène.

8. Procédé selon la revendication 7, dans lequel le mélange contient de 2 à 10% en poids de propane.

9. Procédé selon l'une quelconque des revendications précédentes, dans lequel la fraction appauvrie en oléfine est amenée à un vapocraqueur.
